# FASCICULE DE BREVET EUROPEEN

(11) **EP 1 641 807 B1**
(45) Date de publication et mention de la délivrance du brevet: **18.06.2008**
(21) Numéro de dépôt: 04767508.7
(22) Date de dépôt: 29.06.2004
(51) Int. Cl.: C07F 9/12

(54) **COMPOSES AROMATIQUES COMME INHIBITEURS DES ALDOLASES**
AROMATISCHE VERBINDUNGEN ALS ALDOLASE INHIBITOREN
AROMATIC COMPOUNDS AS ALDOLASE INHIBITORS

(30) Priorité: 02.07.2003 FR 0308042
(43) Date de publication de la demande: 05.04.2006
(73) Titulaire: UNIVERSITE PAUL SABATIER TOULOUSE III, 31062 Toulouse Cédex 4 (FR); CENTRE NATIONAL DE LA RECHERCHE SCIENTIFIQUE (CNRS), 75016 Paris (FR); Valorisation-Recherche, Société en Commandite, Montréal QC H3X 2H9 (CA)
(72) Inventeur: DAX, Chantal, F-31200 Toulouse (FR); BLONSKI, Casimir, F-31520 Ramonville (FR); AZEMA, Laurent, F-31190 Auterive (FR); SYGUSCH, Jurgen, Montreal, Quebec H4A 2W8 (CA)
(74) Mandataire: Cabinet BARRE LAFORGUE & associés
(86) Numéro de dépôt international: PCT/FR2004/001664
(87) Numéro de publication internationale: WO 2005/012313

(56) Documents cités:
- BLONSKI C, ET AL.: "Inhibition of rabbit muscle aldolase by phosphorylated aromatic compounds" BIOCHEM. J., vol. 323, 1997, pages 71-77, XP002266231 cité dans la demande
- GEFFLAUT T, ET AL.: "Class I Aldolases" PROG. BIOPHYS. MOLEC. BIOL., vol. 63, no. 3, 1995, pages 301-340, XP002266232 cité dans la demande

## Description

L'invention concerne de nouveaux composés inhibiteurs de la glycolyse, dont l'action se manifeste par une inhibition des aldolases. L'invention concerne aussi leur procédé de synthèse et leurs applications -notamment dans la fabrication de médicaments pour le traitement du cancer.

La glycolyse représente un mécanisme complexe qui fait intervenir une cascade de dix enzymes différentes pour assurer la dégradation anaérobie du glucose avec libération d'énergie emmagasinée sous forme d'ATP (adénosine-5'-triphosphate).

Parmi les dix enzymes prenant part à la glycolyse, les aldolases (fructose-1,6-bisphosphate aldolases), enzymes homotétramériques, interviennent dans une étape importante : le clivage du fructose-1,6-bisphosphate (Fru(1,6)*P₂*) en deux trioses phosphates, le dihydroxyacétone phosphate (DHAP) et le glycéraldéhyde-3-phosphate (GAP). Le clivage du Fru(1,6)*P₂* par les aldolases s'effectue selon un mécanisme multi-étapes définissant une pluralité d'intermédiaires réactionnels bien identifiés.

Les aldolases sont essentielles dans la voie de la glycolyse, et par conséquent précieuses pour la synthèse du pyruvate et de l'ATP.

Les aldolases se répartissent selon deux classes (Ruther et al., 1964, Fed. Proc. Fed. Am. Soc. Exp. Biol., 23:1248-1257). Les aldolases d'une classe se distinguent de celles de l'autre classe notamment par leurs origines, mais aussi par leur mode de fonctionnement. Les aldolases de classe I, présentes dans l'espèce animale, les plantes supérieures et certains parasites, fixent le Fru(1,6)*P₂* en formant une imine protonée (ou base de Schiff) entre un résidu de lysine (lys-229) du site actif et le substrat. Les aldolases de classe II, quant à elles, sont spécifiques des organismes procaryotes. Elles nécessitent la présence, au niveau de leur site catalytique, d'un ion métallique bivalent pour fixer et stabiliser le substrat sous forme énolate, avant clivage.

Parmi les inhibiteurs des aldolases de classe 1 connus à ce jour, on peut citer des inhibiteurs compétitifs, notamment ceux décrits dans Blonski C. et al., 1997 (Biochem. J., 323:71-77) : des dérivés phosphates de l'hydroquinone (HQN-*P₂*), du résorcinol (RSN-*P₂*) et le benzaldéhyde-4-phosphate (HBA-*P*). Testés sur l'aldolase de muscle de lapin, ces inhibiteurs présentent, respectivement une constante de dissociation (ou constante d'inhibition) *Kᵢ* de l'ordre de 135, 40 et 500 µM. On connaît aussi des composés phosphatés de constante d'inhibition *Kᵢ* de l'ordre de 30-40 µM (Hartman et al., 1965, Biochemistry, 4:1068-1075).

On peut aussi citer le 2-hydoxybenzaldéhyde-4-phosphate (Blonski C. et al, 1997, Biochem. J., 323:71-77) un inhibiteur réversible dépendant du temps de constante d'inhibition globale *Kᵢ** de 35 ± 5 µM, capable de réduire 80 % de l'activité des aldolases pour une concentration d'inhibiteur de l'ordre de 200 µM.

A l'instar de ces composés, les autres inhibiteurs des aldolases connus à ce jour (Gefflaut T. et al., 1995, Prog. Biophys. Mol. Biol., 63(3):301-340), et les inhibiteurs de la glycolyse en général, présentent une activité inhibitrice faible, à moins de les utiliser à très forte concentration, ce qui limite considérablement leur champ d'application. Aussi, leur application à faible concentration, par exemple à des concentrations compatibles avec des doses thérapeutiques, est difficilement envisageable.

Or, dans bien des applications, et en particulier dans le domaine médical, une inhibition significative de la glycolyse est recherchée et ce pour des concentrations d'inhibiteur suffisamment faibles pour éviter tout effet nocif pour le patient. A ce titre, on connaît une approche, désignée par approche GRH ("Glycerol rescued hypoglycemia"), imaginée pour le traitement de certains cancers et de certaines tumeurs solides (Mc Carty M.F., 2001, Med. Hypothèses, 56:286-289). Son fondement repose en particulier sur deux constats.

En premier lieu, il a été montré que les tumeurs solides et les cancers létaux qui se développent dans des tissus où règne une faible concentration en oxygène (conditions d'hypoxie), possèdent un phénotype faisant intervenir la glycolyse comme voie unique de production d'énergie. Il a été également montré que les cellules de ce type se caractérisent aussi par une absence d'activité de la glycérol kinase, une enzyme particulière qui permet aux cellules saines d'utiliser le glycérol en remplacement du glucose.

L'approche GRH propose donc d'inhiber la voie de la glycolyse dans toutes les cellules du patient tout en lui dispensant une dose suffisante de glycérol en guise de source unique d'énergie. Les cellules cancéreuses dont la glycolyse représente la voie primordiale de synthèse d'ATP se trouvent ainsi "affamées" ne pouvant utiliser ni le glucose, ni le glycérol comme source d'énergie alternative au glucose. Les cellules saines, quant à elles, grâce à l'activité glycérol-kinase et d'autres enzymes de la partie inférieure de la glycolyse (celle des trioses) peuvent utiliser le glycérol pour la synthèse du pyruvate. On affame donc les cellules cancéreuses tout en assurant aux tissus sains un apport énergétique convenable.

Cependant, à ce jour, encore aucun traitement anticancéreux empruntant l'approche GRH n'a pu être mis en oeuvre en raison de l'absence d'inhibiteurs suffisamment efficaces de la glycolyse, et plus particulièrement d'inhibiteurs d'une des enzymes de la partie supérieure de la glycolyse (celle des hexoses).

De même, la glycolyse représentant la seule voie de synthèse énergétique pour de nombreux parasites, le blocage de la glycolyse peut être également une approche très prometteuse pour le traitement de nombreuses pathologies d'origine parasitaire.

Il existe ainsi actuellement une réelle nécessité de pouvoir disposer d'au moins un inhibiteur efficace de la glycolyse, notamment dans la perspective de surmonter le cancer ainsi que certaines maladies parasitaires, ou dans le cadre d'éradiquer tout organisme pour lequel la glycolyse représente une voie de synthèse d'énergie principale, voire essentielle.

La présente invention vise à pallier les lacunes précédemment signalées en proposant de nouveaux composés chimiques ayant la capacité d'inhiber la glycolyse et en particulier l'activité des aldolases de classe I.

L'invention vise plus particulièrement à proposer des composés qui par leur efficacité d'inhibition des aldolases peuvent être utilisés à titre de médicaments, c'est-à-dire efficacement et ceci à des doses thérapeutiques -notamment dans le cadre de traitements du cancer-.

L'invention a également pour objectif de proposer des applications de ces composés à titre d'agents inhibiteurs de la glycolyse, à l'exception des applications thérapeutiques. Par extension, l'invention tend à proposer de nouveaux médicaments, notamment pour le traitement du cancer et/ou des maladies parasitaires.

Enfin, l'invention a pour objectif de proposer un procédé de synthèse des composés selon l'invention, de mise en oeuvre à la fois simple, quantitative et peu coûteuse, en d'autres termes, un procédé compatible avec les contraintes économiques d'une production à l'échelle industrielle.

Dans tout le texte, on adopte la terminologie suivante :
- maillon aromatique : cycle conjugué d'un noyau aromatique dans lequel les électrons libres peuvent se déplacer d'un atome de carbone à un autre sans se lier à aucun d'eux (délocalisation des électrons par effet de résonance), et d'un atome de carbone d'un maillon à un atome de carbone d'un autre maillon ; les noyaux aromatiques de type naphtalène mis en oeuvre dans l'invention présentent ainsi une alternance de liaisons simples (liaisons σ) et doubles (liaisons π), conduisant à un système d'électrons π fortement délocalisés entre les deux maillons aromatiques formant ces noyaux,
- mimétique du groupement phosphate : substituant d'un composé chimique, autre qu'un groupement phosphate, adapté pour améliorer la biodisponibilité du composé chimique au niveau de sa cible protéique (l'aldolase) dans le cadre de l'invention ; les mimétiques du groupement phosphate peuvent être de deux types : des groupements protecteurs enzymolabiles ou des analogues stables du phosphate,
- groupement protecteur enzymolabile : mimétique du groupement phosphate adapté pour permettre la diffusion passive du composé à travers des systèmes membranaires cellulaires ou parasitaires, selon une méthode de masquage de charges puis pour subir, à l'intérieur de la cellule ou du parasite, une transformation (ou déprotection) enzymatique de façon à pouvoir générer un groupement phosphate ou un analogue stable du phosphate,
- analogue stable du phosphate : mimétique du groupement phosphate qui par comparaison à un groupement phosphate présente une stabilité améliorée vis-à-vis des conditions physiologiques -notamment pH et/ou environnement enzymatique-.

L'invention concerne un composé aromatique selon la revendication 1.

Bien que les mécanismes réels de l'inhibition des aldolases de classe I ne soient pas définitivement élucidés spécifiquement pour chacun des composés de l'invention, les travaux des inventeurs sur certains de ceux-ci ainsi que les travaux de modélisation du site actif de différentes aldolases de classé I, permettent d'envisager une inhibition efficace de ces aldolases grâce à des composés qui répondent à la définition précédente.

L'invention a ainsi permis de montrer que la présence simultanée d'un groupement phosphate, d'un groupement hydroxyle et d'un groupement aldéhyde, greffés à une structure chimique de géométrie plane telle qu'un noyau aromatique de type naphtalène, est nécessaire pour générer une bonne reconnaissance et une affinité appréciable avec le site actif des aldolases.

Le groupement phosphate est essentiel à la reconnaissance de l'inhibiteur par l'aldolase. Le groupement phosphate s'insère à l'intérieur de la poche de reconnaissance de l'enzyme et permet au composé aromatique d'adopter une bonne orientation à l'intérieur du site actif. Le composé aromatique expose alors les groupements aldéhyde et phénol à bonne distance d'au moins un des trois résidus de lysine (Lys-107, Lys-146, Lys-229) du site actif, de façon à former avec celui-ci, à pH physiologique, une liaison stable de type imine protonée ou base de Schiff. L'exigence de la disposition du groupement phosphate sur un maillon aromatique autre que celui occupé par l'aldéhyde et l'hydroxyle, a ainsi une grande importance. En effet, un noyau aromatique de type naphtalène permet de positionner l'hydroxyle et l'aldéhyde à une distance du groupement phosphate particulièrement appropriée pour une bonne affinité du composé vis-à-vis des aldolases ; la distance séparant le groupement phosphate de l'aldéhyde et de l'hydroxyle conduit à une reconnaissance du groupement phosphate par les aldolases et à la stabilisation du composé dans le site actif, par le biais d'un des trois résidus de lysine, avec une très faible contrainte exercée sur le composé et donc une faible dépense d'énergie.

D'autre part, les positions respectives de l'aldéhyde et de l'hydroxyle par rapport au phosphate jouent un rôle important dans la sélectivité d'une aldolase particulière pour un composé bien défini selon l'invention.

Enfin, la présence des groupements hydroxyle et aldéhyde sur deux atomes de carbone adjacents d'un même maillon aromatique est primordiale et gouverne la formation de cette liaison particulière au niveau du site actif des aldolases de classe I. Le groupement hydroxyle joue vraisemblablement un rôle de catalyseur acide lors de la déshydratation de la carbinolamine intermédiaire et intervient ensuite vraisemblablement dans la stabilisation de la base de Schiff protonée, générant ainsi une inhibition efficace quasi-irréversible, voire irréversible.

Un noyau aromatique de type naphtalène sur lequel sont greffés les groupements phosphate, aldéhyde et phénol à des positions particulières, permet également et avantageusement de définir un composé à géométrie plane conformationnellement plus contrainte que les substrats des aldolases (Fru(1,6)*P*₂, GAP, DHAP), et de dimensions adaptées, respectant à la fois les contraintes d'accessibilité du composé au site actif des aldolases, et d'encombrement stérique à ce niveau.

De tels composés aromatiques sont donc susceptibles d'occuper le site actif des aldolases de classe I de sorte que la fixation et le clivage du Fructose-1,6-bisphosphate puissent être empêchés, et *in fine,* de façon à entraîner le blocage de la glycolyse. De tels composés aromatiques sont ainsi susceptibles d'inhiber de façon efficace les aldolases de cellules eucaryotes et/ou de parasites, et donc de bloquer la glycolyse des cellules eucaryotes et/ou parasitaires.

Cependant, lorsqu'une inhibition intracellulaire et/ou intraparasitaire est souhaitée, l'état d'ionisation du groupement phosphate, nécessaire à la reconnaissance et à l'encrage d'un composé selon l'invention au site actif des aldolases, engendre un problème pour le passage de ces inhibiteurs à travers la membrane cellulaire, se traduisant par une mauvaise biodisponibilité de ces composés d'où une faible activité globale.

Une première approche permettant d'améliorer la biodisponibilité d'un composé phosphate, consiste à protéger les groupements phosphates de façon à masquer temporairement ces charges -notamment au moyen de groupements protecteurs enzymolabiles-, et ainsi permettre leur passage de façon passive à travers des membranes cellulaires.

Par ailleurs, le pH, notamment dans les tissus vivants, cellules et parasites, peut être à l'origine d'un clivage spontané du groupement phosphate. De même, de nombreuses enzymes -notamment les phosphatases-sont capables de cliver la liaison cycle aromatique-phosphate. Aussi, pour les applications sur des tissus, cellules, parasites vivants, il est préférable de substituer ces groupements phosphates par des mimétiques, notamment des analogues stables du phosphate.

Avantageusement, un mimétique du groupement phosphate est choisi parmi:
- un groupement protecteur enzymolabile adapté pour permettre audit composé aromatique un passage passif à travers des systèmes membranaires cellulaires et/ou parasitaires et pour pouvoir générer, une fois à l'intérieur d'une cellule ou d'un parasite, la formation d'un groupement phosphate ou d'un analogue stable du groupement phosphate,
- un analogue stable du phosphate adapté pour préserver le composé aromatique d'une déphosphorylation spontanée et/ou enzymatique
- notamment par les phosphatases cellulaires et/ou parasitaires-.

A titre d'exemples non limitatifs de composés selon l'invention susceptibles d'inhiber les aldolases, on peut citer des composés qui, selon la nomenclature officielle, peuvent être désignés par : le 5-formyl-6-hydroxy-2-naphtylphosphate, le 6-formyl-5-hydroxy-2-naphtylphosphate, le 4-formyl-5-hydroxy- 1 -naphtylphosphate, le 5-formyl-4-hydroxy-1-naphtyl-phosphate, le 4-formyl-3-hydroxy-2-naphtylphosphate, le 3-formyl-4-hydroxy-2-naphtylphosphate, le 3-formyl-2-hydroxy-1-naphtylphosphate, le 2-formyl-3-hydroxy-1-naphtylphosphate, ou un dérivé de ces derniers obtenu par la substitution du groupement phosphate par un mimétique du groupement phosphate selon l'invention.

Avantageusement, un composé aromatique selon l'invention a pour formule générale : 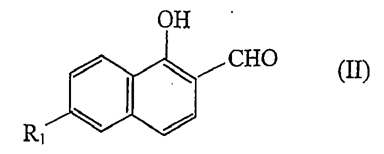 où R₁ est un groupement phosphate ou un mimétique du groupement phosphate.

Lorsque R₁ est un groupement phosphate, le composé aromatique selon la formule générale (II) est alors désigné, conformément à la nomenclature officielle, par le 5-formyl-6-hydroxy-2-naphtylphosphate (désigné par la suite par FHN-*P*). Aussi, un composé selon l'invention et de formule générale (II) peut être le 5-formyl-6-hydroxy-2-naphtylphosphate ou un dérivé de ce dernier, obtenu par une substitution du groupement phosphate par un mimétique selon l'invention.

Des études d'interaction du FHN-*P* avec les aldolases de classe I, plus particulièrement avec l'aldolase de muscle de lapin, ont été réalisées par spectroscopie UV/visible de différence et par spectrométrie de masse électrospray. Ces études ont été complétées à la fois par des analyses de cinétiques enzymatiques et des expériences utilisant des aldolases mutées.

Sur l'aldolase de muscle de lapin, l'invention a permis de mettre en évidence la formation d'une base de Schiff entre le groupement aldéhyde du composé aromatique selon l'invention et l'enzyme. Elles ont également permis de montrer l'implication du résidu Lys-107 dans la formation de cette base de Schiff. Enfin, l'inhibition de l'aldolase de muscle de lapin par le FHN-*P* a été définie comme étant une inhibition réversible dépendante du temps ("slow-binding inhibition"). Ce type d'inhibition, outre le fait de présenter un haut degré de sélectivité, se caractérise par une fixation lente de l'inhibiteur à l'enzyme qui peut être aussi efficace qu'une inhibition réalisée par un inhibiteur de type irréversible chimiquement réactif (Morrison et Walsch, 1988, Adv. Enzymol. Relat. Aeras Mol. Biol., 61:201-301). L'inhibition de l'aldolase de muscle de lapin par le FHN-*P* se caractérise par une constante globale d'inhibition *Kᵢ** de l'ordre de 25 nM. Ainsi, ce composé représente à ce jour l'inhibiteur réversible dépendant du temps le plus puissant vis-à-vis des aldolases de classe I. Qui plus est, comme toute inhibition impliquant la formation d'une base de Schiff entre inhibiteur et un résidu de lysine, cette inhibition peut être rendue irréversible par un traitement au borohydride de sodium du complexe inhibiteur-enzyme.

Il est à noter qu'un composé tel que le 2-(4-formyl-3-hydroxyphényl)-1-éthylène dihydrogène phosphate peut être considéré dans l'inhibition des aldolases comme un équivalent de FHN-*P*. En effet, ce composé répond, de façon très comparable au FHN-P, aux conditions nécessaires à l'inhibition des aldolases, précédemment exposées. La présence du groupement phosphate de ce composé permet tout d'abord une reconnaissance par les aldolases. Ce composé tout comme le FHN-*P* s'oriente alors à l'intérieur du site actif et, compte tenu de la distance séparant le groupement phosphate des groupements aldéhyde et phénol, très sensiblement identique à celle des groupements de FHN-*P*, il expose au proche voisinage d'un des résidus de lysine ses groupements aldéhyde et phénol.

Avantageusement, un groupement protecteur enzymolabile selon l'invention est un groupement de nature à être déprotégé par une(des) estérase(s) intracellulaire(s).

Avantageusement, et selon l'invention ce groupement protecteur enzymolabile présente la formule générale suivante : 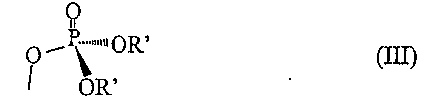 où R' peut être choisi parmi un des groupements suivants :

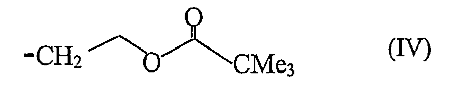

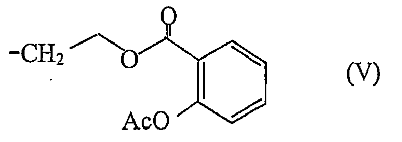

Un composé aromatique selon l'invention présente ainsi un groupement protecteur enzymolabile, dit de type oxyméthylester. Les mécanismes de déprotection d'un tel groupement enzymolabile sont notamment décrits par Farquhar et al., 1983 (J. Pharm. Sci., 72:324-325), et par Srivastva et al., 1984 (Bioorg. Chem., 12:118-124).

Avantageusement, selon une autre variante, un groupement protecteur enzymolabile selon l'invention, de nature à être déprotégé par des estérases intracellulaires, présente la formule générale suivante : 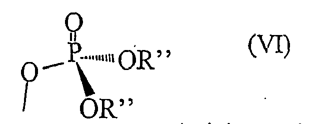 où R" peut être choisi parmi un des groupements suivants :

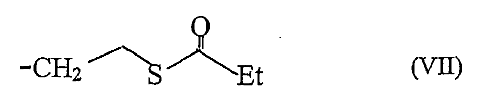

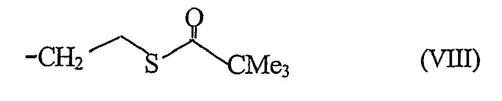

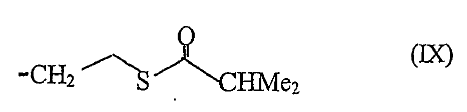

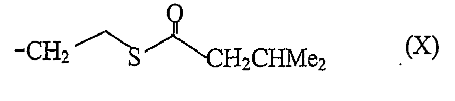

Bien que les groupements protecteurs enzymolabiles de type thioester font intervenir pour leur déprotection les mêmes enzymes que pour les groupements protecteurs enzymolabiles de type oxyméthylester, le mécanisme réactionnel est cependant bien différent et est notamment décrit par Lefebvre et al., 1995 (J. Med. Chem., 38:3941-3950).

Selon une variante de réalisation en matière de masquage temporaire de charge, un groupement protecteur enzymolabile selon l'invention est susceptible d'être déprotégé par une(des) réductase(s) intracellulaire(s).

Avantageusement, selon cette variante de réalisation, un groupement protecteur enzymolabile répond à la formule générale suivante : 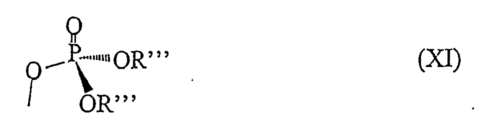 où R"' peut être choisi parmi un des groupements suivants :

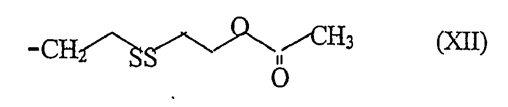

Les mécanismes de déprotection d'un tel groupement protecteur enzymolabile ont été décrits par Puech et al., 1993 (Antiviral Res., 22:155-174).

Il est bien entendu possible de pallier l'état ionisé du groupement phosphate de certains composés aromatiques selon l'invention par des techniques autres que le masquage de charges par des groupements protecteurs enzymolabiles. Selon l'application envisagée (sur des aldolases extraites ou non de cellules), on pourra préférer avoir recours à un encapsulage particulier (forme gallénique), ou à une technique d'administration particulière (méthodes d'injections), en complément ou en remplacement des groupements protecteurs enzymolabiles, et notamment ceux précédemment cités.

Avantageusement, un analogue stable du phosphate selon l'invention peut être choisi parmi l'un des groupements suivants :
- le méthylènephosphonate,
- le difluorométhylènephosphonate,
- le monofluorométhylènephosphonate,
   notamment décrits dans Nieschalk J. et al., 1996 (Tetrahedron Lett., 52(1):165-176).

Avantageusement et selon l'invention, un atome d'hydrogène d'au moins un des atomes de carbone d'au moins un des maillons aromatiques est substitué par un substituant, dit substituant hydrophobe adapté pour améliorer l'hydrophobicité globale du composé. La présence d'un tel groupement facilite l'accès d'un composé aromatique selon l'invention au site actif, relativement hydrophobe, des aldolases. Il convient en outre de choisir un groupement compatible avec la synthèse du composé selon l'invention et de tenir compte des contraintes d'encombrement stérique au niveau du site actif des aldolases.

Avantageusement, chacun des substituants hydrophobes selon l'invention est un groupement alkyle de chaîne principale comprenant au maximum trois atomes de carbone.

L'invention concerne en outre un composé aromatique selon l'invention pour ses utilisations comme agent inhibiteur de l'activité des aldolases de classe I, et par extrapolation, comme agent inhibiteur de la glycolyse, chez les organismes eucaryotes. Avantageusement, l'utilisation d'un composé aromatique se fait dans un cadre non thérapeutique.

Avantageusement et selon l'invention, un composé aromatique utilisé à cette fin est susceptible de présenter une constante d'inhibition *Kᵢ* des aldolases inférieure à 25 µM, notamment inférieure à 50 nM -typiquement de l'ordre de 25 nM-.

Avantageusement et selon l'invention, un composé aromatique utilisé à cette fin est susceptible d'inhiber au moins une aldolase de façon irréversible ou quasi irréversible.

L'invention s'étend à un procédé d'inhibition d'aldolases extracellulaires ou intracellulaires, avantageusement à l'exception des aldolases intracellulaires de cellules vivantes non isolées d'un corps humain ou d'un corps animal ou d'un embryon humain, dans lequel on met ces aldolases au contact d'au moins un composé aromatique selon l'invention, au moins en quantité suffisante pour induire un effet notable sur l'activité des aldolases.

Avantageusement, un procédé selon l'invention est appliqué à l'inhibition de la glycolyse cellulaire.

Avantageusement, un procédé selon l'invention peut être appliqué pour enrayer le développement d'une cellule cancéreuse.

L'invention s'étend aussi à un médicament comprenant un composé aromatique selon l'invention.

Dans la lutte contre le cancer, différentes approches thérapeutiques ont été imaginées (chimiothérapie, radiothérapie, chirurgie,...) et de nombreux traitements sont aujourd'hui utilisés. Toutefois, à ce jour, il n'existe aucun traitement totalement efficace contre les cancers. Le choix des traitements actuels se fait généralement en fonction du type de tissu et des organes atteints, ainsi que du degré de diffusion et de la malignité. Cependant, beaucoup de traitements sont mal supportés à plus ou moins long terme par les patients. De ce fait, une approche multi-thérapeutique est généralement prescrite, d'où la nécessité de pouvoir disposer de différents traitements pour pouvoir soigner un même type de cancer et qu'il sera possible de prescrire alternativement à un patient. La possibilité de pouvoir mettre en oeuvre l'approche GRH donnerait lieu à de nouveaux traitements et ainsi un grand progrès dans la lutte contre le cancer. Un composé aromatique selon l'invention permet de mettre en oeuvre très favorablement l'approche GRH.

L'invention s'étend ainsi également à l'utilisation d'un composé aromatique selon l'invention pour la fabrication d'un médicament pour le traitement du cancer -notamment pour la fabrication d'un médicament pour le traitement du cancer s'effectuant selon l'approche GRH-.

L'invention s'étend aussi à un procédé de synthèse d'un composé aromatique selon l'invention.

Selon un premier aspect de l'invention, le procédé permet la synthèse d'un 1-hydroxy-2-naphtaldéhyde phosphorylé sur un atome de carbone du deuxième maillon aromatique.

Avantageusement, un procédé de synthèse selon l'invention comprend une étape de phosphorylation d'un composé 2-naphtaldéhyde dihydroxylé, hydroxylé sur l'atome de carbone 1 du premier maillon aromatique et sur un des atomes de carbone du second maillon aromatique, ladite phosphorylation correspondant à une substitution du groupement hydroxyle du second maillon aromatique par un groupement phosphate.

Avantageusement et selon l'invention, l'étape de phosphorylation est initiée par la technique du triéthyl phosphite, pyridine et iode, dans une solution de CH₂Cl₂/THF (Stowel J.K. et al., 1995, Tetrahedron Lett., 36:182-185 ; Ladame S. et al., 2001, Phosphorus, Sulfur and Silicon, 174:37-47).

Avantageusement et selon l'invention, la phosphorylation est effectuée sur le 1,6-dihydroxy-2-naphtaldéhyde pour aboutir, après déprotection (Page P. et al., 1999, Bioor. Med. Chem., 7:1403-1412), au 5-formyl-6-hydroxy-2-naphtylphosphate (FHN-*P*).

L'invention concerne aussi un composé aromatique inhibiteur d'aldolase, un procédé d'inhibition des aldolases et de la glycolyse, ainsi qu'un médicament, caractérisés en combinaison par tout ou partie des caractéristiques mentionnées ci-dessus ou ci-après.

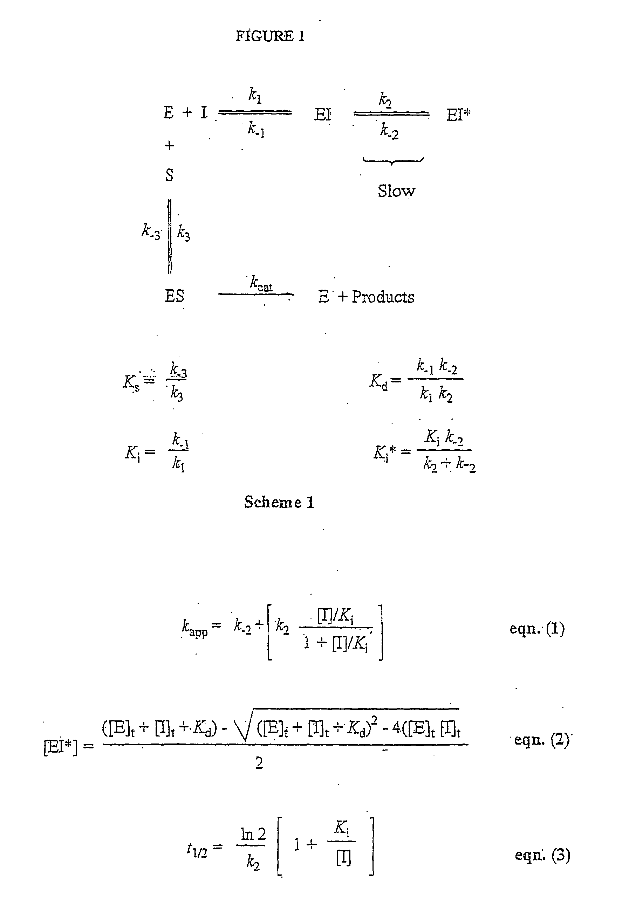

D'autres-buts, caractéristiques et avantages de l'invention apparaissent à la lecture des exemples qui suivent. Les figures 1 à 5 illustrent les résultats obtenus dans ces exemples.

### Méthodes de synthèse et d'analyse physico-chimique

Toutes les réactions sensibles à l'humidité ont été réalisées sous atmosphère d'argon dans des conditions anhydres.

Le tétrahydrofurane (THF) et l'éther éthylique sont distillés sur sodium/benzophénone. Le dichlorométhane est distillé sur P₂O₅. La pyridine et la triéthylamine sont distillées sur de la potasse. Le méthanol est distillé en présence d'une quantité faible de sodium. Les solvants pour les chromatographies liquides à haute pression (HPLC) sont utilisés sans autres purifications.

L'évolution des réactions a été suivie par chromatographie sur couche mince (CCM) sur plaque de silice (Merck 60-F254) en utilisant des systèmes d'éluants appropriés. Les produits ont été révélés par irradiation UV (λ=254 nm et 366 nm) ou par utilisation de vapeur d'iode ou de révélateur spécifique comme le zinzade (molybdate d'ammonium) pour les produits phosphorylés, ou le révélateur de Pancaldi contenant 5% de sulfate de cérium (IV), une solution aqueuse d'acide sulfurique à 6% et 8% de molybdate d'ammonium.

Les produits ont été purifiés par chromatographie flash sur gel de silice (Merck-60, 230-400 mesh) ou bien par chromatographie liquide à haute pression (HPLC) préparative sur colonne phase inverse C18 (Macherey-Nagel polygoprep C18, 12-25 µm, 60A) avec des systèmes d'éluants appropriés.

Les spectres RMN ¹H, ¹³C et ³¹P ont été enregistrés sur des spectromètres Bruker AC 200 ou AC 250. Les déplacements chimiques (δ) sont exprimés en ppm, à partir du tétraméthylsilane pour les noyaux ¹H et ¹³C, à partir de l'acide phosphorique pour le noyau ³¹P. Les valeurs des constantes de couplages sont exprimées en Hz. Les abréviations suivantes ont été utilisées : s, singulet ; se singulet élargi ; d, doublet ; t, triplet ; q, quadruplet et m, multiplet.

Les spectres de masse (DCI) sont réalisés sur un spectromètre quadripolaire Nermag R10-10.
- Les analyses élémentaires ont été effectuées sur un appareil Eager 200.

### EXEMPLE 1 : Synthèse du 5-formyl-6-hydroxy-2-naphtylphosphate (FHN-P) :

Le 1,6-dihydroxy-2-naphtaldéhyde (0,21 g ; 1,1 mmol), préalablement synthétisé (Jonhson W.S.J. et Shelberg W.E., 1945, J. Am. Chem. Soc., 67:1745-1753 ; Jonhson W.S.J. et al., 1944, J. Am. Chem. Soc., 66:218-222 ; Bilger C. et al., 1987, Eur. J. Med. Chem., 22:363-365), est mis en solution dans du CH₂Cl₂ (34 ml). On ajoute de la pyridine anhydre (0,1 ml ; 1,24 mmol) maintenue à 0°C. On ajoute alors un mélange de iode (0,1 g ; 1,2 mmol) et de triéthyle phosphite (0,23 ml ; 1,32 mmol) dissous dans du CH₂Cl₂ (10 ml). L'ensemble est maintenu sous agitation à 0°C pendant 1 heure puis laissé à température ambiante. De l'eau (20 ml) est ajoutée. La phase organique est lavée avec de la saumure (solution d'eau saturée en NaCl) puis séchée sur du MgSO₄. On supprime le solvant à pression réduite. Après chromatographie flash (CH₂Cl₂), une huile de couleur jaune est obtenue (0,11 g ; 31 %) : le 1-hydroxy-2-naphtaldéhyde-6-diéthylphosphate.

Du bromotriméthylsilane (0,15 ml ; 1,1 mmol) est ajouté lentement sous agitation à la solution de 1-hydroxy-2-naphtaldéhyde-6-diéthylphosphate (0,054 g ; 0,17 mmol) précédemment obtenue, en solution dans du CH₂Cl₂ anhydre (200 µl) sous atmosphère d'azote. Le mélange obtenu est agité pendant 3 heures à température ambiante. Du Et₂O/H₂O (10:1) est alors ajouté et la phase organique obtenue est lavée à l'eau. Le pH de la phase aqueuse est ajusté à 7,6 avec de la soude 1 M. La solution est lyophilisée pour aboutir à une poudre blanche (0,050 g ; 96%) de 5-formyl-6-hydroxy-2-naphtylphosphate (FHN-*P*) sous forme de sel de sodium.
¹H NMR (250 MHz, D₂O) : δ7,11 (d, *³J_{H3-H4}*=8,50 Hz, 1H, H3) ; 7,34 (d, *³J_{H7-H8}*=8,00 Hz, 1H, H7) ; 7,41 (se, 1H, H5) ; 8,24 (d, *³J_{H4-H3}*=8,50 Hz, 1H, H4) ; 9,84 (d, *³J_{HS-H7}*=8,00 Hz, 1H, H8) ; 9,91 (s, 1H, CHO).
¹³C NMR (50 MHz, D₂O) : δ114,73 (C2) ; 116,93-(C10) : 118,32 (d, *³J_{C-P}*=3,57 Hz, C5) ; 120,13 (C8); 123,36 (d, *³J_{C-P}*=5,00 Hz, C7) ; 123,42 (C9) : 126,72 (C3) ; 128,31 (C4) ; 142,35 (C1) ; 158,49 (d, *²J_{C-P}=*6,00 Hz, C6) ; 1-98,32 (CHO). ³¹P NMR (81 MHz, D₂O) : δ0,56.
Spectrométrie de masse (FAB) : 267,λₘₐₓ^{H}₂^{O} (pH 7,6) : 392 nm (ε5100 M⁻¹·cm⁻¹), 277 nm (ε4650 M⁻¹·cm⁻¹).

### EXEMPLE 2 : Activité inhibitrice de l'aldolase de muscle de lapin (RM) par FHN-P

### • Méthode d'analyse :

L'activité aldolase a été mesurée au moyen d'un système de tests enzymatiques couplés : le système triose-phosphate isomérase et glycérol-3-phosphate déshydrogénase (TIM/GDH) en suivant l'oxydation du NADH (Boehringer-Mannheim, France), à 340 nm, au moyen d'un spectrophotomètre SAFAS thermostaté à 25°C. Les tests ont été initiés par addition du substrat (le Fru (*1,6*)*P₂* ; 1 mM en concentration finale), pour compléter une solution d'aldolase réalisée dans un tampon triéthanolamine (tampon TEA) (100 mM TEA/HCl ; 50 mM NaCl ; 1 mM EDTA, pH 7,6 ; de force ionique 0,15), 0,42 mM de NADH, et contenant les deux systèmes enzymatiques (10 µg/ml de GDH et 1 µg/ml de TIM) pour un volume final de 1 ml. La construction, la surexpression et la purification de l'aldolase recombinante ont été réalisées selon Morris A.J. et al., 1993, (J. Biol. Chem., 265:1095-1100) et Berthiaume L. et al., 1993 (J. Biol. Chem., 268:10826-10835).

Le taux de clivage du substrat est déterminé en mesurant la décroissance de l'absorbance/minute à 340 nm. L'aldolase est dialysée toute la nuit à 4°C contre un tampon TEA avant utilisation. La concentration en protéine est déterminée par spectrophotométrie en utilisant comme coefficient d'extinction ε₂₈₀=0,91 ml.mg⁻¹.cm⁻¹. La concentration de sous-unités est déterminée en se basant sur un poids moléculaire de 159 000 pour un tétramère d'aldolase.

### - Méthode cinétique :

Dans le cas présent, l'inhibition réversible dépendante du temps ("Slow binding inhibition") implique la formation rapide d'un équilibre entre l'enzyme E et l'inhibiteur I, suivie d'un complexe initial EI qui subit une isomérisation lente et réversible vers un complexe EI* cinétiquement plus stable, comme montré dans le schéma réactionnel et dans les équations réactionnelles présentés à la Figure 1, où *Kᵢ** représente la constante globale d'inhibition, *Kᵢ* la constante de dissociation du complexe de Michaelis EI, rapidement formé, et *K_{d}* la constante de dissociation du complexe EI*.

### - Caractérisation de l'inhibition réversible dépendante du temps :

En absence du substrat la constante de vitesse apparente d'ordre 1 *(kₐₚₚ)* décrivant la formation du complexe EI* est définie par l'équation eqn.(1) (Figure 1) (Morrison et Walsh, 1988, Adv. Enzymol. Relat. Aeras Mol. Biol, 61:201-300).

La valeur de *kₐₚₚ* reflète la cinétique de saturation lorsque la concentration d'inhibiteur [I] croît, elle varie entre des limites basse et haute, respectivement, de *k₂* et *k₂*+*k₋₂.*

A l'équilibre, lorsque [EI*]>[EI], on utilise l'équation eqn.(2) (Figure 1), où [E]ₜ et [I]ₜ représentent les concentrations initiales, respectivement, de l'enzyme et de l'inhibiteur ; et *K_{d}* est la constante de dissociation du complexe EI* (Segal I.H., 1975, Enzyme kinetics : Behavior and Analysis of Steady-State and Rapid Equilibrium Enzyme Systems, Willey-Interscience, New-York).

Dans la situation extrême où *k₋₂* tend vers zéro, un inhibiteur réversible dépendant du temps peut s'apparenter à un inhibiteur irréversible dirigé contre le site actif. Dans cette situation, l'équation eqn.(3) est utilisée pour ce type d'inhibition (Meloche H.P., 1967, Biochemistry, 6:2273-2280). Le temps de demi-inactivation de l'enzyme (t_{1/2}) se définit comme étant une fonction de la concentration de l'inhibiteur réciproque et est représentée graphiquement par une droite coupant l'axe des ordonnées en ln(2/*k₂*) et coupant l'axe des abscisses en -1/*Kᵢ*.

### • Protocole expérimental :

L'aldolase native (5 µM de sous-unités) est incubée en présence de FHN-*P* (0,05-1 mM) dans le tampon TEA. L'activité enzymatique est analysée en fonction du temps avec des aliquots de 10 µl.

La cinétique d'inactivation suit une cinétique de pseudo premier ordre et les paramètres cinétiques *Kᵢ* et *k₂* sont déterminés en utilisant l'équation eqn.(3). Pour déterminer le taux de réactivation, l'aldolase (25 µM de sous-unités dans le tampon TEA) est incubée avec 500 µM de FHN-*P* jusqu'à obtenir 90% d'inactivation. L'excès d'inhibiteur est enlevé par ultrafiltration en utilisant une membrane (Millipore) PM-30. Le complexe enzyme-inhibiteur est incubé dans le tampon TEA (15 µM de sous-unités en concentration finale) contenant l'hexitol-*P₂* (10 mM). Des aliquots (10 µl) sont prélevés à différents temps pour mesurer l'activité aldolase.

Les contrôles consistent à répéter le protocole sans FHN-*P*. Le processus de réactivation est analysé comme si c'était une réaction d'ordre 1.

### - Suivi par spectroscopie UV/visible de différence.

Les spectres d'absorption sont mesurés en utilisant un spectrophotomètre Cary 1E Varian thermostaté à 25°C. Le même tampon TEA est utilisé pour les titrations et les analyses d'activité. Les spectres d'absorption sont mesurés par deux méthodes différentes, notamment décrites dans Gefflaut T. et al., 1986 (Bioorg. Med. Chem., 4:2043-2054) et Blonski C. et al., 1997 (Biochem. J., 323:71-77).

Pour la méthode A, les spectres d'absorption sont enregistrés en fonction temps, soit entre 250 et 500 nm, soit à des longueurs d'onde correspondant à l'absorption maximale ou minimale. Les mesures sont initiées par addition de FHN-*P* à différentes concentrations finales .(0,1-1 mM) dans le tampon TEA renfermant une concentration fixe d'aldolase (10 µM de sous-unités). Les spectres d'absorption mesurés du complexe enzymatique sont corrigés avec l'absorbance du tampon, de FHN-*P*, et de l'enzyme (mesurées séparément). Les spectres d'absorption de différence ainsi obtenus sont utilisés pour déterminer la valeur des constantes définissant la formation du complexe aldolase-inhibiteur.

La méthode B est utilisée pour le titration du FHN-*P* par l'acide aminocaproique. Pour chaque analyse au tampon TEA renfermant une concentration fixe de FHN-*P* (10 µM), l'acide aminocaproique est ajouté pour obtenir différentes concentrations finales (0,01-0,2 M). Les spectres d'absorption de différence, correspondant à la formation d'une base de Schiff, sont enregistrés à différents intervalles de temps, puis corrigés avec l'absorbance du tampon, de l'acide aminocaproique, et de FHN-*P* (mesurées séparément).

La constante de vitesse apparente d'ordre 1 *(kₐₚₚ)* et les variations d'absorption maximales limites (ΔAₘₐₓ) sont obtenues pour chaque test en appliquant les données d'absorption dépendantes du temps à l'équation cinétique d'ordre 1 (ou à l'ensemble des deux mécanismes cinétiques d'ordre 1). La constante de dissociation (*K_{d}*) de la base de Schiff formée entre le FHN-P et l'acide aminocaproique est obtenue à partir des différences d'absorption déterminées à l'équilibre en utilisant l'équation eqn.(2). La valeur de la constante de dissociation (*Kᵢ*) du complexe EI rapidement formé et celle de la constante *k₂* correspondant à la formation lente du complexe EI* proviennent de l'analyse des valeurs des constantes d'ordre 1 selon les équations eqn.(1) ou eqn.(3).

Tous les tests de spectroscopie UV/visible de différence utilisant des aldolases mutants (10 µM de sous-unités) sont menés en utilisant la méthode A.

### - Suivi par spectrométrie de masse électrospray (ESI/MS)

Les spectres de masse électrospray sont obtenus en mode positif sur un quadripôle Finnigan Mat TSQ 700. Les aldolases (50 µM de sous-unités dans le tampon TEA) sont incubées avec 50 µM de FHN-*P* jusqu'à atteindre 60±5 % d'inactivation. L'excès d'inhibiteur est supprimé par ultrafiltration au tampon acétate d'ammonium (10 mM, pH 5,5). Les échantillons sont préparés à environ 10 pmol/µl dans H₂O/méthanol (1:1, v/v) avec une concentration finale de 1 mM d'acétate d'ammonium et 0,5% d'acide acétique. Les échantillons sont infusés à l'intérieur de la source du spectromètre avec un débit continu de 4 µl/min.

### • Résultats

### - Inhibition réversible dépendant du temps de l'aldolase par FHN-P.

L'incubation de l'aldolase de muscle de lapin avec le FHN-*P* conduit à une perte de l'activité enzymatique selon une cinétique d'ordre 1.

Le substrat Fru(1,6)*P₂* et le substrat analogue l'hexitol-*P₂*, un puissant inhibiteur compétitif des aldolases, montrent une protection de l'enzyme contre l'inactivation par le FHN-*P* (Tableau 1), correspondant à une inhibition se produisant au niveau du site actif de l'enzyme.

Les conditions expérimentales et les mesures effectuées, reprises dans le tableau 1 ci-après, sont les suivantes : l'aldolase native (RM) de muscle de lapin (0,2 mg/ml) est incubée en présence d'une concentration fixe de FHN-*P* (250 µM) dans le tampon TEA (volume final de 1 ml ; pH 7,6) avec ou sans Fru(1,6)*P₂* ou hexitol-*P₂* (1 mM) ; l'activité enzymatique des aliquotes est analysée après 80 minutes d'incubation.

Par comparaison, dans les mêmes conditions expérimentales (décrites dans le Tableau 1), l'analogue non-phosphorylé du FHN-*P* (1,6-dihydroxy-2-naphtaldéhyde ou DHNA, à 250 µM) est totalement inactif suggérant fortement l'implication fonctionnelle du groupement phosphate dans 1a fixation de FHN-*P* au site actif de l'enzyme. Les paramètres cinétiques dérivent des constantes apparentes d'ordre 1 *(kₐₚₚ*) mesurées pour différentes concentrations d'inhibiteur (0,01-1,5 mM) selon l'équation eqn.(3) et sont repris dans le Tableau 2.

**Tableau 1**

| Conditions | Activité spécifique (Unité/mg) |
|---|---|
| RM seul | 12,5±0,5 |
| RM + FHN-*P* | 2,8±0,3 |
| RM + FHN-*P* + Fru(1,6)*P₂* | 5,2±0,4 |
| RM + FHN-*P* + hexitol-*P₂* | 9,9±0,5 |
| RM + DHNA | 12,5±0,5 |

**Tableau 2**

| Paramètres | Valeur |
|---|---|
| *Kᵢ*(*µ*M) | 250±100 |
| *k₂* (min⁻¹) | 0,07±0,01 |
| *k₋₂*(min⁻¹) | 13±7.10⁻⁶ |
| *Kᵢ** (nM) | 40±20 |

L'activité enzymatique est récupérée pour partie en incubant les enzymes inactivées dans une solution dépourvue de FHN-*P* et contenant une concentration saturante en hexitol-*P₂* (10 mM). La valeur de la constante d'ordre 1 (*k₋₂*=1,3±7,10⁻⁵min⁻¹) reflétant le recouvrement de l'activité enzymatique est d'environ quatre ordres inférieurs à la constante d'inactivation k₂.

FHN-P se comporte donc comme un inhibiteur réversible dépendant du temps. D'après les valeurs exposées dans le Tableau 2, la constante globale de dissociation *Kᵢ** peut être estimée à 40±20 nM. Par ailleurs, l'activité enzymatique ne peut être restaurée après un traitement du complexe enzyme-inhibiteur par le borohydride de sodium, ce qui suggère que le mécanisme d'inhibition par FHN-*P* engendre la formation d'une base de Schiff avec un résidu de lysine du site actif.

### - Interaction du FHN-P avec l'acide aminocaproique

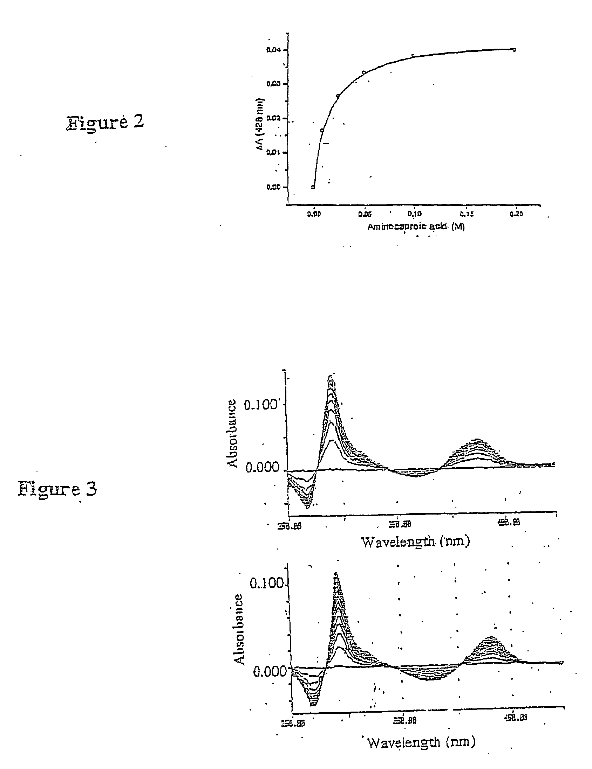

Le FHN-*P* et le groupe ε-amino des résidus de lysine de l'aldolase ont été pris comme modèle pour la formation de la base de Schiff ; la réaction de FHN-*P* (10µM) avec l'acide aminocaproique (0,1-1 mM) comme référence, et a été suivie par spectroscopie UV/visible de différence. La réaction modèle, représentée à la Figure 3 (partie supérieure), se caractérise par des changements d'absorbance en UV/visible de différence correspondant à deux maxima à 425 nm (Δε 3730 ± 200 M⁻¹.cm⁻¹) et à 291 nm (Δε 13080 ± 700 M⁻¹.cm⁻¹), ce qui peut être attribué à la formation d'une base de Schiff. Cette réaction modèle se caractérise également par deux minima à 367 nm (Δε-740 ± 50 M⁻¹.cm⁻¹) et à 267 nm (Δε-5650 ± 300 M⁻¹.cm⁻¹) résultant de la perte de FHN-P à l'intérieur-du complexe covalent. La présence des points isosbestiques à 388, 343 et 276 nm indique que les intermédiaires ne s'accumulent pas et qu'il n'y a pas non plus de réaction secondaire. Les changements d'absorbance différentielle à l'équilibre deviennent saturants lorsqu'on augmente les concentrations en acide aminocaproique (Figure 2) et la cinétique de réaction pour chacune de ces concentrations s'apparente à celle d'un mécanisme réactionnel de pseudo premier ordre et correspond à une constante d'ordre 2, *k_{2nd}* de 0,12 ± 0,01 M⁻¹.min⁻¹ à 25°C. La valeur de la constante de dissociation, *K_{d}*, du complexe FHN-*P*-acide aminocaproique est de 16 ± 3 mM (Figure 2).

### - Interaction entre FHN-P et l'aldolase de muscle de lapin

Lorsque le FHN-*P* (0,1-1 mM, de concentration finale) est ajouté à une solution contenant l'aldolase native (10 µM de sous-unités), lé spectre UV/visible de différence résultant présente des maxima à 431 et 293 nm, des minima à 376 et 268 nm, et des points isosbestiques à 401 et 279 nm (Figure 3, partie inférieure). Les légères différences dans les positions de bandes avec la réaction modèle peuvent être attribuées à des différences d'environnement protéique de FHN-*P* par rapport au système modèle. L'évolution des spectres de différences d'absorbance coïncide avec deux mécanismes d'ordre 1 distincts. La phase de cinétique rapide se manifeste par un changement d'absorbance plus important et un comportement saturant à concentration élevée d'inhibiteur, corrélée à une perte totale d'activité de l'enzyme. Une phase de cinétique lente est également observée lorsque les analyses sont menées à des concentrations saturantes d'hexitol-*P₂* (10 mM). Cette phase de cinétique lente, qui correspond à une constante trente fois plus faible et qui contribue à un quart de la variation finale d'absorbance n'est pas liée à l'inhibition observée. A ces concentrations saturantes, les coefficients d'absorption molaire relatifs au FHN-*P* lié sont calculés en supposant que les quatre sites actifs des aldolases sont occupés: Δε₄₃₁ = 3600 ± 200 M⁻¹·cm⁻¹ ; Δε₃₇₆ = -2140 ± 110 M⁻¹·cm⁻¹ ; Δε₂₉₃ = 12480 ± 650 M⁻¹·cm⁻¹ et Δε₂₆₈ = 5080 ± 250 M⁻¹·cm⁻¹.

La stoechiométrie de la liaison de FHN-*P* à l'aldolase pour une inhibition maximale est cohérente avec les absorbances moléculaires différentielles provenant du complexe acide aminocaproique-FHN-*P*, qui prédit au complexe EI* ayant 3,9-4,2 moles de FHN-*P* liés par mole d'aldolase tétramérique.

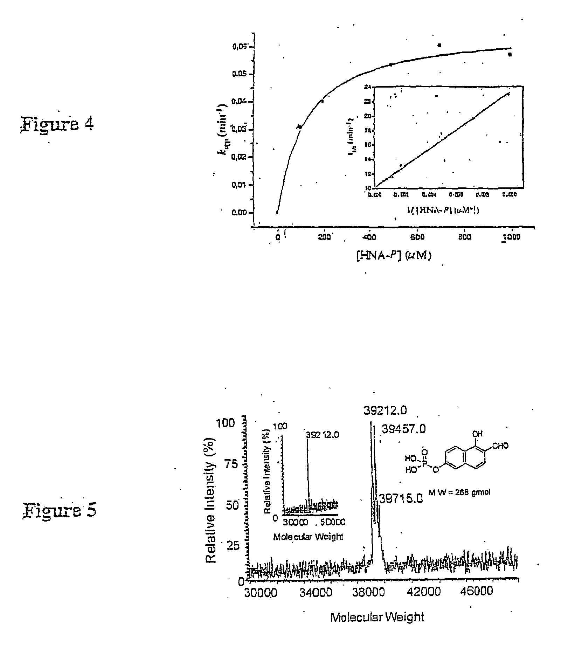

Les paramètres cinétiques, *Kᵢ* et *k₂* caractérisant la formation du complexe EI* sont déterminés à partir des données exposées à la Figure 4 selon les équations eqn.(1) ou eqn.(3) et conduisant aux valeurs de *Kᵢ* = 125 ± 25 µM, k₂ =0,067 ± 0,004 min-l et t½ ~ 6 min.

La constante globale d'inhibition *Kᵢ** calculée à partir de ces derniers résultats correspond à 25 ±15 nM.

### - Analyse du complexe aldolase-FHN-P par spectrométrie de masse électrospray.

L'analyse en spectrométrie de masse électrospray d'un complexe aldolase-FHN-*P* a conduit à des masses moléculaires correspondant à : un monomère d'aldolase (39212 Da) ; un complexe binaire entre une sous-unité d'aldolase et le FHN-*P* (39460 Da), sous forme de base de Schiff, et des traces de complexe tertiaire entre une sous-unité d'aldolase et 2 molécules de FHN-*P* (39712 Da) (Figure 5).

### - Interaction entre les mutants de l'aldolase RM et FHN-P

Les résidus de lysine 107, 146 et 229 sont localisés au niveau des sites actifs de l'aldolase et sont ainsi des condidats pour réaliser l'inactivation via la formation d'une base de Schiff avec le FHN-*P*. Pour identifier quelle lysine est responsable des absorbances différentielles en présence de FHN-*P*, des mutations ponctuelles de ces résidus de lysine ont été réalisées et nommées : K107M (Research Collaboratory for Structural Bioinformatics Protein Databank), K146M (Blonski C. et al., 1997, Biochem. J. 323:71-77) et K229M (Research Collaboratory for Structural Bioinformatics Protein Databank). La comparaison des coordonnées atomiques de K107M, K229M, et de l'enzyme native, montre que ces mutants de structure sont isomorphes par rapport à l'enzyme sauvage (les déviations standard ("rms déviations") des coordonnées atomiques par rapport à l'aldolase natif est de 0,41 Å et 0,48 Å respectivement pour les structures K107M et K229M). La spectroscopie UV/visible de différence (méthode A) a été utilisée pour examiner la formation du complexe enzymatique pour chaque mutation ponctuelle en présence d'une concentration fixe de FHN-*P* (300 µM). L'analyse des données obtenues à 431 nm est reportée au Tableau 3.

**Tableau 3**

| Aldolase | Activité spécifique (unités/mg) | Activité spécifique résiduelle (%) | *kₐₚₚ* (min⁻¹) | ΔA relative à 431 nm (%) |
|---|---|---|---|---|
| Sauvage | 12,5 | nd (non détecté) | 0,045±0,0025 | 100 |
| | | | 0,0015±0,0003 | 25 |
| K229M | 3.10⁻⁶ | nd | 0,040±0,0025 | 95 |
| | | | 0,0015±0,0003 | 25 |
| K107M | 0,7 | 95 | 0,0015±0,0005 | 30 |
| K146M | 2.10⁻³ | nd | 0,005±0,0005 | 98 |
| | | | 0,0015±0,0003 | 25 |

Les spectres de différence observés pour le mutant K229M sont identiques à ceux observés avec l'aldolase sauvage. L'évolution de la réaction peut être justifiée par deux mécanismes d'ordre 1 distincts. Les paramètres cinétiques et le niveau de complexe EI* formé sont identiques à ceux obtenus avec l'enzyme sauvage, et excluent la Lys-229 comme résidu de lysine directement responsable de la formation de la base de Schiff.

Par comparaison, les spectres de différences du mutant KI07M et de FHN-*P* montrent une phase de cinétique lente dont la constante et l'absorbance maximale sont identiques aux valeurs de la phase lente observée pour l'enzyme sauvage. L'absence de phase rapide ajoutée à l'absence d'inhibition de l'enzyme prouve l'implication de la Lys-107 dans le phénomène d'inhibition réversible dépendante du temps.

La réaction du mutant K146M avec FHN-*P* a pour résultat un changement global d'absorbance à l'équilibre, comparable avec ce qui est observé pour l'aldolase sauvage (Tableau 3). La phase de la cinétique associée à l'inhibition enzymatique, bien que correspondant à un mécanisme d'ordre 1, diffère de ce qui est observé dans le cas de l'aldolase sauvage par une formation très lente de la base de Schiff. La substitution de la Lys-147 par une méthionine engendre donc une baisse significative du taux de complexes EI* formés, mais ne change pas la capacité du mutant à former le complexe. Ainsi, la Lys-146 n'est pas requise pour la formation de la base de Schiff mais facilite considérablement celle-ci.

## Revendications

1. Composé aromatique comprenant un groupement aldéhyde, un groupement phénol et un groupement phosphate ou un groupement mimétique du groupement phosphate, **caractérisé :**
- **en ce que** son noyau aromatique comprend deux maillons aromatiques de type benzénique,
- et **en ce qu'**il répond à la formule générale suivante : 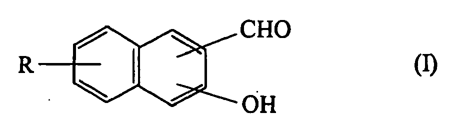
dans laquelle :
• le groupement aldéhyde (-CHO) et le groupement phénol (-OH) sont fixés à deux atomes de carbone adjacents d'un même maillon aromatique, dit premier maillon aromatique,
• et fixé à un atome de carbone d'un deuxième maillon aromatique dit second maillon aromatique, R est un groupement phosphate ou un groupement mimétique du groupement phosphate de formule générale ; 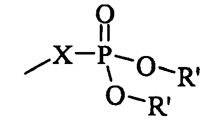 dans lequel ;
- X est choisi dans le groupe formé d'un atome d'oxygène, de
- CH₂-, de -CHF-, et de -CF₂-,
- R' est choisi dans le groupe formé de l'atome d'hydrogène et des substituants (IV), (V), (VII), (VIII), (IX), (X) et (XII).

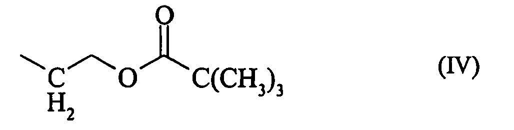

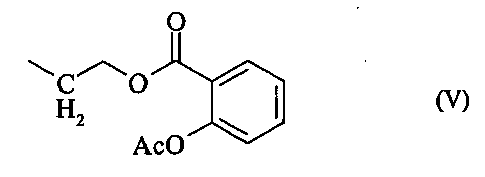

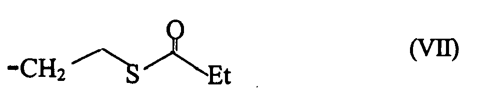

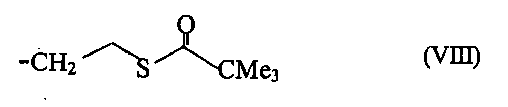

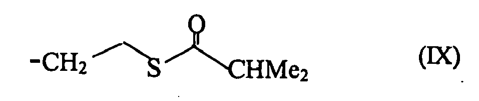

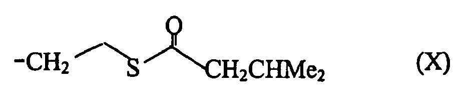

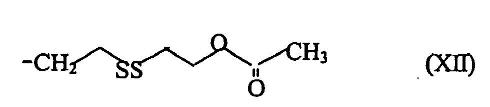

2. Composé aromatique selon la revendication 1, **caractérisé en ce qu'**il a pour formule générale ; 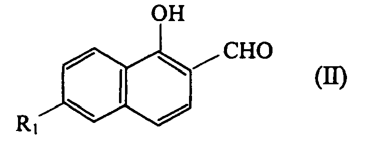
• où R₁ est un groupement de formule générale ; 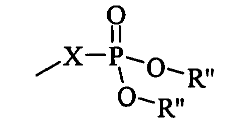
dans lequel ;
- X est choisi dans le groupe formé d'un atome d'oxygène, de
- CH₂-, de -CHF-, et de -CF₂-,
- R" est choisi dans le groupe formé de l'atome d'hydrogène et des substituants (IV), (V), (VII), (VIII), (IX), (X) et (XII).

3. Composé aromatique selon l'une des revendications 1 ou 2, **caractérisé en ce qu'**un atome d'hydrogène d'au moins un des atomes de carbone d'au moins un des maillons aromatiques est substitué par un substituant, dit substituant hydrophobe, adapté pour améliorer l'hydrophobicité globale du composé aromatique.

4. Composé aromatique selon la revendication 3, **caractérisé en ce que** le(les) substituant(s) hydrophobe(s) est(sont) un(des) groupement(s) alkyle(s) de chaîne principale comprenant au maximum trois atomes de carbone.

5. Composé aromatique selon l'une des revendications 1 à 4, pour ses utilisations non-thérapeutiques comme agent inhibiteur de l'activité des aldolases de classe I.

6. Composé aromatique selon la revendication 5, **caractérisé en ce qu'**il présente une constante d'inhibition *Kᵢ* inférieure à 25 µM, notamment inférieure à 50 nM, typiquement de l'ordre de 25 nM.

7. Composé aromatique selon la revendication 5, **caractérisé en ce qu'**il est susceptible d'inhiber au moins une aldolase de façon irréversible ou quasi irréversible.

8. Procédé d'inhibition d'aldolases extracellullaires ou intracellulaires, à l'exception des aldolases intracellulaires de cellules vivantes non isolées d'un corps humain ou animal, ou d'un embryon humain, **caractérisé en ce qu'**on met lesdites aldolases au contact d'au moins un composé aromatique selon l'une des revendications 1 à 7, au moins en quantité suffisante pour induire un effet notable.

9. Procédé selon la revendication 8, appliqué à l'inhibition de la glycolyse cellulaire.

10. Procédé selon la revendication 8 ou 9, appliqué pour enrayer le développement d'une cellule cancéreuse.

11. Médicament comprenant un composé aromatique selon l'une des revendications 1 à 7.

12. Utilisation d'un composé selon l'une des revendications 1 à 7, pour la fabrication d'un médicament pour le traitement du cancer.

13. Utilisation selon la revendication 12, pour la fabrication d'un médicament pour le traitement du cancer s'effectuant selon l'approche GRH.

14. Procédé de synthèse d'un composé 1-hydroxy-2-naphtaldéhyde phosphorylé sur un atome de carbone du deuxième maillon aromatique, selon la revendication 1, dans lequel on réalise une étape de phosphorylation d'un composé 2-naphtaldéhyde dihydroxylé, hydroxylé sur l'atome du carbone 1 du premier maillon aromatique et sur un des atomes de carbone du second maillon aromatique, ladite phosphorylation correspond à une substitution du groupement hydroxyle du second maillon aromatique par un groupement phosphate.

15. Procédé de synthèse selon la revendication 14, **caractérisé en ce que** la phosphorylation est initiée par la technique du triéthyl phosphite, pyridine et iode, dans une solution de CH₂Cl₂/THF.

16. Procédé de synthèse selon la revendication 14 ou 15, **caractérisé en ce qu'**il est effectué sur le 1,6-dihydroxy-2-naphtaldéhyde pour conduire au 5-formyl-6-hydroxy-2-naphtylphosphate.

## Claims

1. An aromatic compound comprising an aldehyde group, a phenol group and a phosphate group or a phosphate-mimetic group, **characterised:**
- **in that** its aromatic nucleus comprises two aromatic chain links of the benzene type,
- and **in that** it corresponds to the following general formula: 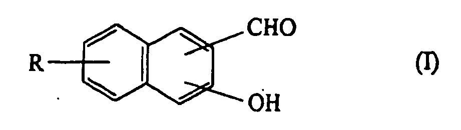
in which:
• the aldehyde group (-CHO) and the phenol group . (-OH) are attached to two adjacent carbon atoms in the same aromatic chain link, referred to as the first aromatic chain link,
• and attached to a carbon atom in a second aromatic chain link, referred to as the second aromatic chain link, R is a phosphate group or a phosphate-mimetic group of the general formula: 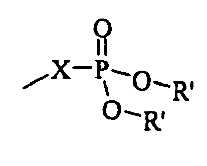
in which:
- X is selected from the group consisting of an oxygen atom, -CH₂-, -CHF- and -CF₂-,
- R' is selected from the group consisting of the hydrogen atom and the substituents (IV), (V), (VII), (VIII), (IX), (X) and (XII).

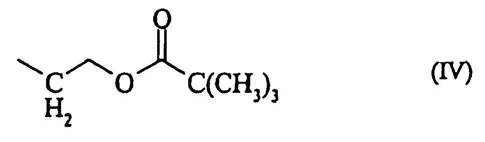

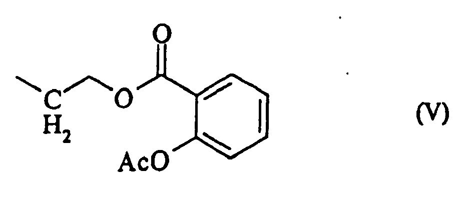

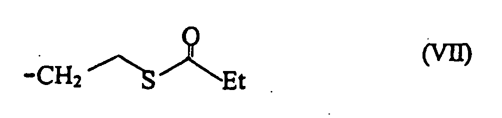

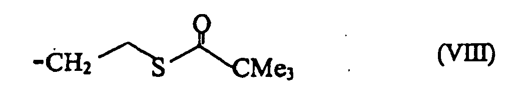

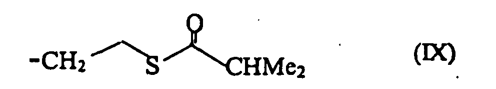

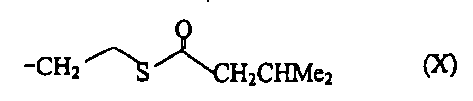

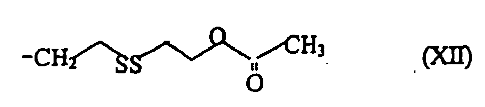

2. The aromatic compound according to claim 1, **characterised in that** it has the following as its general formula: 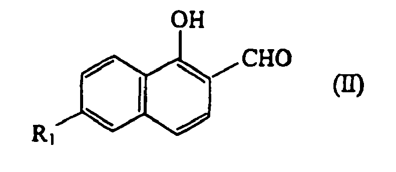
• where R₁ is a group with the general formula: 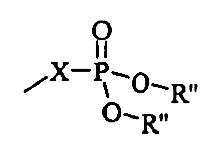
in which:
- X is selected from the group consisting of an oxygen atom, -CH₂-, -CHF- and -CF₂-,
- R" is selected from the group consisting of the hydrogen atom and the substituents (IV), (V), (VII), (VIII), (IX), (X) and (XII).

3. The aromatic compound according to one of claims 1 or 2, **characterised in that** a hydrogen atom of at least one of the carbon atoms of at least one of the aromatic chain links is substituted by a substituent, referred to as the hydrophobic substituent, suitable for improving the overall hydrophobicity of the aromatic compound.

4. The aromatic compound according to claim 3, **characterised in that** the hydrophobic substituent(s) is/are an alkyl group or groups with a main chain comprising a maximum of three carbon atoms.

5. The aromatic compound according to one of claims 1 to 4, for its non-therapeutic uses as an inhibitor of class I aldolase activity.

6. The aromatic compound according to claim 5, **characterised in that** it has an inhibition constant Kᵢ of less than 25 µM, particularly less than 50 nM, typically of the order of 25 nM.

7. The aromatic compound according to claim 5, **characterised in that** it is capable of inhibiting at least one aldolase irreversibly or almost irreversibly.

8. A process for the inhibition of extracellular or intracellular aldolases, with the exception of intracellular aldolases of living cells not isolated from a human or animal body or a human embryo, **characterised in that** said aldolases are brought into contact with at least one aromatic compound according to one of claims 1 to 7, in a quantity at least sufficient to bring about a notable effect.

9. The process according to claim 8, applied to the inhibition of cellular glycolysis.

10. The process according to claim 8 or 9, applied to stop the development of a cancerous cell.

11. A medicament comprising an aromatic compound according to one of claims 1 to 7.

12. The use of a compound according to one of claims 1 to 7 for the manufacture of a medicament for the treatment of cancer.

13. The use according to claim 12 for the manufacture of a medicament for the treatment of cancer carried out in accordance with the GRH approach.

14. A process for the synthesis of a 1-hydroxy-2-naphthaldehyde compound phosphorylated on a carbon atom in the second aromatic chain link, according to claim 1, in which a phosphorylation step is performed on a dihydroxylated 2-naphthaldehyde, hydroxylated on the carbon atom 1 of the first aromatic chain link and on one of the carbon atoms of the second aromatic chain link, said phosphorylation corresponding to a substitution of the hydroxyl group of the second aromatic chain link by a phosphate group.

15. The synthesis process according to claim 14, **characterised in that** the phosphorylation is initiated by the triethyl phosphite, pyridine and iodine technique in a solution of CH₂Cl₂/THF.

16. The synthesis process according to claim 14 or 15, **characterised in that** it is performed on 1,6-dihydroxy-2-naphthaldehyde to lead to 5-formyl-6-hydroxy-2-naphthyl phosphate.

## Patentansprüche

1. Aromatische Verbindung mit einer Aldehydgruppe, einer Phenolgruppe und einer Phosphatgruppe oder einer mimetischen Gruppe der Phosphatgruppe, **dadurch gekennzeichnet:**
- **dass** ihr aromatischer Kern zwei aromatische Kettenglieder vom Benzoltyp umfasst,
- und **dass** sie der folgenden allgemeinen Formel unterliegt: 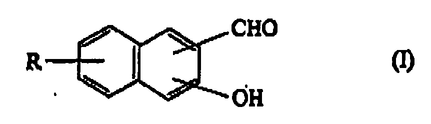 in der:
* die Aldehydgruppe (-CHO) und die Phenolgruppe (-OH) an zwei Kohlenstoffatomen fixiert sind, die einem und demselben, als erstes aromatisches Kettenglied bezeichneten aromatischen Kettenglied benachbart sind,
* und an einem als zweites aromatisches Kettenglied bezeichnetes Kohlenstoffatom einem zweiten aromatischen Kettenglied fixiert sind, R ist eine Phosphatgruppe oder eine mimetische Gruppe der Phosphatgruppe der allgemeinen Formel: 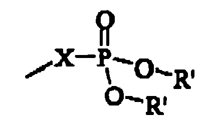 in der:
- X aus der aus einem Sauerstoffatom, aus -CH₂, aus -CHF und aus -CF₂- gebildeten Gruppe ausgewählt wird,
- R' aus der aus dem Wasserstoffatom und den Substituenten (IV), (V), (VII), (VIII), (IX),)X) und (XII) gebildeten Gruppe ausgewählt ist

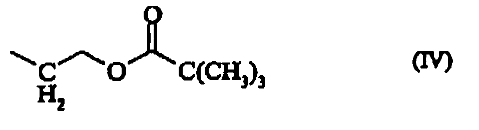

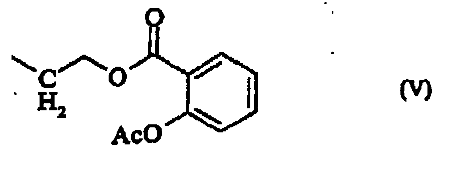

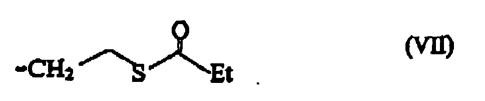

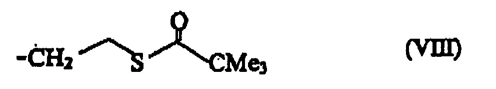

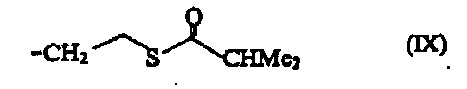

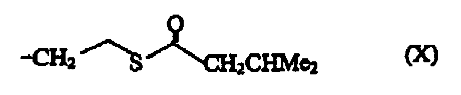

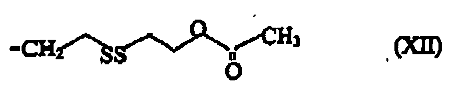

2. Aromatische Verbindung gemäß Anspruch 1, **dadurch gekennzeichnet, dass** sie die folgende allgemeine Formel hat: 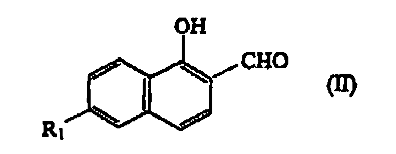
* in der R₁ eine Gruppe mit der allgemeinen Formel 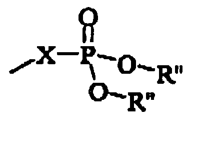 ist, in der:
- X aus der aus einem Sauerstoffatom, aus -CH₂-, aus -CHF- und aus -CF₂- gebildeten Gruppe ausgewählt ist,
- R" aus der aus dem Wasserstoffatom und den Substituenten (IV), (V), (VII), (VIII), (IX), (X) und (XII) gebildeten Gruppe ausgewählt ist.

3. Aromatische Verbindung gemäß Anspruch 1 oder 2, **dadurch gekennzeichnet, dass** wenigstens ein Wasserstoffatom wenigstens eines der Kohlenstoffatome wenigstens eines der aromatischen Kettenglieder durch einen so genannten hydrophoben Substituenten substituiert ist, der geeignet ist, um die globale Hydrophobizität der aromatischen Verbindung zu verbessern.

4. Aromatische Verbindung gemäß Anspruch 3, **dadurch gekennzeichnet, dass** der / die hydrophobe(n) Substituent(en) eine / mehrere Hauptketten-Alkylgruppe(n) ist / sind, die wenigstens drei Kohlenstoffatome umfassen.

5. Aromatische Verbindung gemäß Anspruch 1 bis 4 für ihre nicht therapeutischen Verwendungen als Inhibitorwirkstoff der Aldolaseaktivität der Klasse I.

6. Aromatische Verbindung gemäß Anspruch 5, **dadurch gekennzeichnet, dass** sie eine Inhibitionskonstante Kᵢ von weniger als 25 µM, insbesondere weniger als 50 nM, typischerweise in der Größenordnung von 25 nM aufweist.

7. Aromatische Verbindung gemäß Anspruch 5, **dadurch gekennzeichnet, dass** sie geeignet ist, wenigstens eine Aldolase irreversibel oder praktisch irreversibel zu inhibieren.

8. Extrazelluläres oder intrazelluläres Aldolase-Inhibitionsverfahren, mit Ausnahme der intrazellulären Aldolasen der nicht isolierten lebenden Zellen eines menschlichen oder tierischen Körpers oder eines menschlichen Embryos, **dadurch gekennzeichnet, dass** die genannten Aldolasen mit wenigstens einer aromatischen Verbindung gemäß Anspruch 1 bis 7 in wenigstens ausreichender Menge in Kontakt gebracht werden, um eine bemerkbare Wirkung zu erzielen.

9. Verfahren gemäß Anspruch 8, das auf die Inhibition der zellulären Glykolyse angewendet wird.

10. Verfahren gemäß Anspruch 8 oder 9, das zur Ausmerzung der Entwicklung einer Krebszelle angewendet wird.

11. Medikament mit einer aromatischen Verbindung gemäß Anspruch 1 bis 7.

12. Verwendung einer Verbindung gemäß Anspruch 1 bis 7 für die Herstellung eines Medikaments für die Behandlung von Krebs.

13. Verwendung gemäß Anspruch 12 für die Herstellung eines Medikaments für die Behandlung von Krebs, die gemäß dem Ansatz GRH erfolgt.

14. Syntheseverfahren einer auf einem Kohlenstoffatom des zweiten aromatischen Kettengliedes phosphorylierten Verbindung 1-Hydroxy-2-Naphtaldehyd gemäß Anspruch 1, in dem eine Phosphorylisierungsstufe einer auf dem Atom des Kohlenstoffs 1 des ersten aromatischen Kettengliedes und auf einem der Kohlenstoffatome des zweiten aromatischen Kettengliedes dihydroxylierten, hydroxylierten 2-Naphtaldehyd-Verbindung realisiert wird, wobei die genannte Phosphorylierung einer Substitution der Hydroxylgruppe des zweiten aromatischen Kettengliedes durch eine Phosphatgruppe entspricht.

15. Syntheseverfahren gemäß Anspruch 14, **dadurch gekennzeichnet, dass** die Phosphorylierung durch die Technik des Triethyl-Phosphits, Pyridins und Iods in einer Lösung aus CH₂Cl₂/THF initiiert ist.

16. Syntheseverfahren gemäß Anspruch 14 oder 15, **dadurch gekennzeichnet, dass** es auf dem 1,6-Dihydroxy-2-Naphtaldehyd durchgeführt wird, um zum 5-Formyl-6-Hydroxy-2-Naphtylphosphat zu führen.
